Europäisches Patentamt

European Patent Office

Office européen des brevets

(11) Publication number: **0 459 172 B1**

# EUROPEAN PATENT SPECIFICATION

(45) Date of publication of patent specification: **13.09.95**  (51) Int. Cl.⁶: **C07H  13/06,** C07H 1/06

(21) Application number: **91107146.2**

(22) Date of filing: **03.05.91**

(54) **Process for decolorizing sucrose fatty acid esters.**

(30) Priority: **16.05.90 JP 126229/90**

(43) Date of publication of application:
**04.12.91 Bulletin  91/49**

(45) Publication of the grant of the patent:
**13.09.95 Bulletin  95/37**

(84) Designated Contracting States:
**FR GB NL**

(56) References cited:
**EP-A- 0 346 845
EP-A- 0 424 066
US-A- 4 942 228**

**CHEMICAL ABSTRACTS, vol. 96, No. 21, May
24, 1982, Columbus, Ohio, USA MIT-
SUITOATSU CHEMICALS, INC. "De-
colorizationpurifi- cation of saccharide-fatty
acidesters" page 744, abstract-No. 181 570m**

**ULLMANNS ENCYCLOP DIE DER TECH-
NISCHEN CHEMIE, 4th edition, vol. 24,
1983,Verlag Chemie page 730, column 1,
lines 46- 50**

**ROMPPS CHEMIE-LEXIKON, vol. 4,8th edition,
1985 page 2456, column 1, lines 25,**

(73) Proprietor: **DAI-ICHI KOGYO SEIYAKU CO.,
LTD.
55 Nishishichijo Higashikubo-cho
Shimogyo-ku,
Kyoto-shi,
Kyoto-fu (JP)**

(72) Inventor: **Matsumoto, Shusaku
A-308, 10-1, Fukakusa Hotta-cho,
Fushimi-ku
Kyoto-shi (JP)**
Inventor: **Uesugi, Naoto
19, Nishishichijo,
Higashikubo-cho
Shimogyo-ku,
Kyoto-shi (JP)**
Inventor: **Okamoto, Shogo
19, Nishishichijo,
Higashikubo-cho
Shimogyo-ku,
Kyoto-shi (JP)**

(74) Representative: **Türk, Gille, Hrabal, Leifert
Brucknerstrasse 20
D-40593 Düsseldorf (DE)**

ULLMANNS ENCYCLOP DIE DER TECH-
NISCHEN CHEMIE, vol. 22, 4th edition,
1982,Verlag Chemie page 496, column 1,
lines 11, 12

**Description**

The present invention relates to a process for decolorizing sucrose fatty acid esters (sugar esters).

Sucrose fatty acid esters, which are useful as surface active agents, are prepared industrially at present by either a solvent process wherein sucrose is reacted with a methyl ester of a higher fatty acid having 8 to 22 carbon atoms in the presence of a suitable catalyst in an organic solvent such as dimethylformamide or dimethylsulfoxide, as disclosed in Japanese Patent Publication Kokoku No. 35-13102; or an aqueous medium process wherein sucrose is formed into a molten mixture with a fatty acid salt (soap) using water without using an organic solvent, and is then reacted with a higher fatty acid methyl ester in the presence of a catalyst, as disclosed in Japanese Patent Publication Kokoku No. 51-14485.

In any of these processes, the obtained reaction mixture containing the desired sucrose fatty acid ester contains a colored material such as caramel resulting from sucrose by decomposition thereof as well as other impurities such as unreacted sucrose, unreacted fatty acid methyl ester, a soap, a free fatty acid and the like. The impurities must be removed from the product prior to putting on the market. It is also important to remove the colored material from the product in order to raise the commercial value. However, it is difficult to completely remove the colored material by conventional methods for the purification of sucrose fatty acid esters. In general, sucrose fatty acid esters purified according to conventional methods followed by powdering is more or less tinged with caramel color. No method for decreasing such a color has been proposed.

EP-A-0 346 845 discloses a process for producing a sucrose fatty acid polyester comprising reacting sucrose with fatty acid lower alcohol ester(s) in a solvent in the presence of an alkaline catalyst, wherein said fatty acid lower alcohol ester(s) are employed in an amount at least 5 times by mol as much as said sucrose. The alkaline catalyst component is removed from the reaction product thus obtained and the obtained sucrose fatty acid polyester-containing solution is catalytically treated with activated clay. This process enables the production of a sucrose fatty acid polyester containing an extremely small amount of ash components and having an extremely low color value.

It is primary object of the present invention to provide a process for decolorizing sucrose fatty acid esters, which can provide pure white sucrose fatty acid esters.

The above and other objects of the present invention will become apparent from the description hereinafter.

It has been found that when magnesium oxide and active carbon are added to a solution of sucrose fatty acid esters in organic solvents such as dimethylsulfoxide and dimethylformamide, the colored material efficiently adsorbed thereby, thus the sucrose esters can be decolored without deteriorating the sucrose esters.

In accordance with the present invention, there is provided a process for decolorizing sucrose fatty acid esters which comprises dissolving a sucrose fatty acid ester in an organic solvent capable of dissolving it, and adding magnesium oxide and active carbon to the resulting solution.

The process of the present invention is applicable to both crude sucrose fatty acid esters and purified sucrose fatty acid esters treated by a known method of the purification.

In practicing the process of the present invention, a colored sucrose fatty acid ester is dissolved in an organic solvent. Any organic solvents capable of dissolving the sucrose fatty acid ester can be used in the invention. Representative examples of the organic solvent are, for instance, dimethylformamide, dimethylsulfoxide and propylene glycol. It is preferable that the water content in the organic solvent is as low as possible, especially at most 1 % by weight, in order to prevent sucrose fatty acid esters from being decomposed by hydrolysis action of magnesium oxide. The dissolution of the sucrose ester may be carried out at ordinary temperature or an elevated temperature of not more than 100 °C. The concentration of the sucrose ester is usually from 10 to 30 % by weight.

Magnesium oxide and active carbon are then added to the resulting solution at ordinary temperature or a higher temperature, preferably at an elevated temperature of not more than 100 °C, with agitating the solution. The added magnesium oxide powder and active carbon powder deposite, or float on the surface of the solution by agitating action. The agitation is further continued. The agitation is usually conducted for 10 to 40 minutes. The magnesium oxide is used in an amount of about 10 to about 150 parts by weight per 100 parts by weight of the sucrose fatty acid ester. The active carbon is also used in an amount of about 10 to about 150 parts by weight per 100 parts by weight of the sucrose fatty acid ester.

If the organic solvent solution of the sucrose fatty acid ester contains 1 % by weight or more of water, the sucrose fatty acid ester may be hydrolyzed due to a hydrolysis acceleration action of magnesium oxide, as mentioned above. Fatty acid monoesters of sucrose are hydrolyzed in preference to fatty acid diesters and triesters of sucrose. Accordingly, when the monoesters are desired, it is particularly desirable to maintain the water content in the solution of the sucrose ester below 1 % by weight.

Afer the agitation during which colored material is adsorbed by magnesium oxide and active carbon, the depositing or floating magnesium oxide and active carbon can be easily separated and removed in a usual manner, e.g. filtration or centrifugation, using a usual separation device. The solution from which magnesium oxide and active carbon have been removed, e.g. filtrate, contains the sucrose ester and a decreased amount of the colored material such as caramel. Accordingly, nearly pure white sucrose fatty acid ester is obtained by removing the organic solvent from the solution such as filtrate, for example, by distilling away the organic solvent under reduced pressure. For example, when dimethylsulfoxide (DMSO) is used as the organic solvent, the filtrate is subjected to distillation at a temperature of 80° to 110°C under vacuum of 665 to 1330 Pa ( 5 to 10 Torrs).

If it is desired to further improve the hue of the sucrose ester, the above procedures of the dissolution of sucrose ester, the addition of the decolorizer, the agitation of solution, the separation of the decolorizer and the evaporation of solvent may be completely or partially repeated.

According to the process of the present invention, the decoloration of sucrose fatty acid esters can be easily practiced on an industrial scale without any technical problem, whereby white sucrose fatty acid ester having a high commercial value is obtained.

The present invention is more specifically described and explained by means of the following Examples in which all % are by weight unless otherwise noted. It is to be understood that the present invention is not limited to the Examples.

Example 1

A sucrose fatty acid ester produced by a reaction of sucrose and a fatty acid methyl ester mixture of methyl stearate and methyl palmitate in a ratio of 7 : 3 by weight and having the following composition was decolored as follows:

(Composition of Sucrose Ester)

| Ingredients | Amounts (%) |
|---|---|
| Sucrose fatty acid ester* | 94.6 |
| Unreacted fatty acid methyl esters and others | 0.9 |
| Soap | 3.5 |
| Fatty acid | 1.0 |
| Total | 100.0 |

(Note) * The sucrose ester had a monoester content 70.5 %, a diester content 19.2 % and a triester and higher-ester content 10.3 %.

The sucrose ester to be decolored had a color difference $\Delta E$ of 11.86 (NBS unit: NBS = National Bureau of Standards) measured by a colorimetry color-difference meter (type Z-1001DP made by Nippon Denshoku Kogyo Kabushiki Kaisha). The lower the color difference $\Delta E$, the more white when visually observed. The higher the color difference $\Delta E$, the more the sucrose ester is colored.

To 150 g of dimethylsulfoxide (DMSO) having a water content of 0.6 % was added 25 g of the sucrose fatty acid ester having the above composition and color difference, and the mixture was stirred at 80°C for 10 minutes to dissolve the sucrose ester. To the resulting solution was added 10.0 g of MgO powder and 10.0 g of a commercially available active carbon powder and the mixture was stirred at 80°C for 30 minutes. The mixture was then filtered by a usual filter to remove the powders, and dimethylsulfoxide was distilled away from the filtrate to give a decolored sucrose fatty acid ester powder.

The treated sucrose ester had a color difference $\Delta E$ of 4.42 (NBS unit), and a definite improvement in the hue was also visually confirmed. The monoester content of the treated sucrose ester was 70.6 %, thus it was confirmed that the original sucrose fatty acid ester was scarcely changed.

The results are shown in Table 1.

4

Comparative Example 1

The procedure of Example 1 was repeated except that 20.0 g of magnesium oxide alone was used instead of combination of magnesium oxide and active carbon.

The treated sucrose fatty acid ester had a color difference ΔE of 5.02 (NBS unit). The degree of improvement in the hue was inferior to that in Example 1. The monoester content was scarcely changed.

The results are shown in Table 1.

Comparative Example 2

The procedure of Example 1 was repeated except that 20.0 g of active carbon alone was used instead of a combination of magnesium oxide and active carbon.

The treated sucrose fatty acid ester had a color difference ΔE of 4.81 (NBS unit). The degree of improvement in the hue was inferior to that in Example 1. The monoester content was scarcely changed.

The results are shown in Table 1.

Table 1

| | Decolorizer (g) | | | Treated sucrose fatty acid ester powder | | |
|---|---|---|---|---|---|---|
| | MgO | Active carbon | Total | Monoester content (%) | Color difference ΔE (NBS unit) | Visual estimation |
| Before treatment | - | - | - | 70.56 | 11.86 | tinged slightly with yellow |
| Ex. 1 | 10.0 | 10.0 | 20.0 | 70.60 | 4.42 | decolored to pure white |
| Com. Ex. 1 | 20.0 | 0 | 20.0 | 70.51 | 5.02 | decolored, but inferior to Example 1 |
| Com. Ex. 2 | 0 | 20.0 | 20.0 | 70.53 | 4.81 | decolored, but inferior to Example 1 |

## Claims

1. A process for decolorizing sucrose fatty acid esters which comprises dissolving a sucrose fatty acid ester in an organic solvent capable of dissolving it, and adding magnesium oxide and active carbon to

6

the resulting solution, wherein the water content of said organic solvent is at most 1.0 % by weight.

2. The process of claim 1, wherein said organic solvent is a member selected from the group consisting of dimethylsulfoxide, dimethylformamide and propylene glycol.

3. The process of claim 1, wherein said magnesium oxide is used in an amount of 10 to 150 parts by weight per 100 parts by weight of said sucrose fatty acid ester.

4. The process of claim 1, wherein said active carbon is used in an amount of 10 to 150 parts by weight per 100 parts by weight of said sucrose fatty acid ester.

5. The process of claim 1, wherein said magnesium oxide is in the form of a powder.

**Patentansprüche**

1. Verfahren zur Entfärbung von Sucrose-Fettsäureestern, welches umfaßt Auflösen eines Sucrose-Fettsäureesters in einem organischen Lösungsmittel, in dem er löslich ist, und Zugabe von Magnesiumoxid und Aktivkohle zu der resultierenden Lösung, wobei der Wassergehalt des organischen Lösungsmittels höchstens 1,0 Gew.-% beträgt.

2. Verfahren nach Anspruch 1, wobei das organische Lösungsmittel ein aus der aus Dimethylsulfoxid, Dimethylformamid und Propylenglykol bestehenden Gruppe ausgewähltes ist.

3. Verfahren nach Anspruch 1, wobei das Magnesiumoxid in einer Menge von 10 bis 150 Gew.-Teilen pro 100 Gew.-Teile des Sucrose-Fettsäureesters verwendet wird.

4. Verfahren nach Anspruch 1, wobei die Aktivkohle in einer Menge von 10 bis 150 Gew.-Teilen pro 100 Gew.-Teile des Sucrose-Fettsäureesters verwendet wird.

5. Verfahren nach Anspruch 1, wobei das Magnesiumoxid in Form eines Pulvers vorliegt.

**Revendications**

1. Procédé pour décolorer des esters d'acide gras du saccharose, qui comprend la dissolution d'un ester d'acide gras du saccharose dans un solvant organique capable de le dissoudre, et l'addition d'oxyde de magnésium et de charbon actif à la solution obtenue, dans lequel la teneur en eau dudit solvant organique est d'au plus 1,0 % en poids.

2. Procédé selon la revendication 1, dans lequel ledit solvant organique est un élément choisi dans le groupe constitué du diméthylsulfoxyde, du diméthylformamide et du propylèneglycol.

3. Procédé selon la revendication 1, dans lequel ledit oxyde de magnésium est utilisé en une quantité de 10 à 150 parties en poids pour 100 parties en poids dudit ester d'acide gras du saccharose.

4. Procédé selon la revendication 1, dans lequel ledit charbon actif est utilisé en une quantité de 10 à 150 parties en poids pour 100 parties en poids dudit ester d'acide gras du saccharose.

5. Procédé selon la revendication 1, dans lequel ledit oxyde de magnésium est sous la forme d'une poudre.